# EUROPEAN PATENT APPLICATION

(11) **EP 1 371 727 A1**
(43) Date of publication of application: **17.12.2003**
(21) Application number: 02700672.5
(22) Date of filing: 21.02.2002
(51) Int. Cl.: C12N 15/11, C12N 1/21, C12N 1/19, C12N 5/10, A01K 67/027

(54) **RECOMBINANT GENE CONTAINING INVERTED REPEAT SEQUENCE AND UTILIZATION THEREOF**

(30) Priority: 22.02.2001 JP 2001046089
(71) Applicant: Gencom Corporation, Machida-shi, Tokyo 194-8511 (JP)
(72) Inventor: KATSUKI, Motoya, Minato-ku, Tokyo 106-0032 (JP); ISHIDA, Mitsuyoshi, Shinagawa-ku, Tokyo 140-0011 (JP); KATO, Minoru, Setagaya-ku, Tokyo 158-0084 (JP)
(74) Representative: Sternagel, Fleischer, Godemeyer & Partner Patentanwälte
(86) International application number: JP0201554
(87) International publication number: WO02066638

(57) **Abstract**

The object of the present invention is to improve a method for introducing dsRNA in such a way that RNAi effect is sustained in mammalian (mainly mouse) cells for a long period of time. The present invention provides a recombinant gene which contains inverted repeats of a target gene which can be expressed in mammalian cells.

## Description

### Technical Field

The present invention relates to a recombinant gene containing inverted repeats of a target gene. More particularly, the present invention relates to a recombinant gene into which inverted repeats of a target gene has been incorporated so as to be expressed in mammalian cells. The present invention also relates to a transgenic animal which is obtained by using the recombinant gene.

### Background Art

In the conventional development of pharmaceuticals, research has been conducted in such a manner that a protein (for example, interferon α) which function is obvious is first discovered in a body, and the DNA is then isolated. Recently, techniques for DNA sequencing have been rapidly advanced resulting from the promotion of genome analysis projects. According to a recently developed method for cDNA analysis, DNA is first isolated and sequenced, and subsequently, the function of DNA is predicted based on whether or not the sequence is similar to known sequences which are registered in DNA databases. Further, DNA functions at the protein level and at the individual (mainly mammalian experimental animal) level are actually elucidated in the subsequent stages. The function of DNA at the individual level has been heretofore elucidated by producing a gene knock-out animal and analyzing its phenotype. However, this knock-out technique requires large amounts of labor and time and thus is not practical for the analysis of a large number of pharmaceuticals or candidate proteins which are targets of pharmaceuticals. Accordingly, a method for inhibiting gene functions in an individual animal, which is more effective and simpler than the conventional knock-out technique, is desired.

RNA interference (RNAi) refers to the phenomenon that, when double-stranded RNA (dsRNA) which is prepared by double-stranding a portion of mRNA coding for a part of certain gene (referred to as "target gene") is introduced into a cell, the expression of the target gene is inhibited. In 1998, it was first found in nematodes that the introduction of dsRNA into a living organism showed an inhibitory action on the expression of the gene which was the same as the transduced gene (Fire et al., 1998). Thereafter, the inhibitory action was observed also in: fungi; plants such as *Nicotiana tabaccum* and *Oryza sativa;* planarians; Trypanosomes such as *Trypanosoma brucei* (Ngo, H., Tschudi. C., Gull, K., and Ullu, E.); flies such as Drosophila melanogaster (Kennerdell and Carthew, 1998); and vertebrates such as zebrafish. Thus, RNAi is regarded as a universal phenomenon which is not limited to specific types of species. In mammals, the action is reported in early mouse embryo (Wianny and Zernikca-Goetz, 2000). The molecular mechanism of the inhibitory action of RNAi has not yet been elucidated, but the involvement of genes such as ego-1, mut-2, mut-7, mut-8, mut-9, red-1, rde-2, and rde-4 has been reported based on genetic analyses in nematodes (Grishok et al., 2000).

The technical application of RNAi was established as a gene knock-out technique in nematodes, and has been used as a major means for analyzing genomic functions using the total nucleotide sequence information obtained by the nematode total genome sequencing project (Fraser et al., 2000; Gonczy et al., 2000). In the analysis of genomic function of mammalian species, RNAi is expected to be an efficient method for inhibiting gene expression since it imparts less burden in terms of tune and labor as compared to gene knock-out techniques.

However, up to now, only a non-genetic technique of directly injecting dsRNA has been reported in mammalian experimental animals (for example, mouse) as a method for introducing dsRNA (Wianny and Zernikca-Goetz, 2000), and conventional gene disruption techniques has not completely been replaced. More specifically, when dsRNA is directly injected into a fertilized egg, dsRNA in each cell is diluted upon cell division, thereby deteriorating gene inhibition efficiency.

In nematodes (Travemarkaris et al., 2000) and *Drosophila* (Kennerdell and Carthew, 2000), transgenic animals have been successfully produced wherein a gene having inverted repeats is introduced, which led to the *in vivo* transcription of dsRNA having hairpin structures, and subsequently caused RNAi expressions. Application of such a genetic technique in mammalian species has been expected, but such a technique has not yet been achieved.

### Disclosure of the Invention

The object of the present invention is to improve a method for introducing dsRNA in such a way that RNAi effect is sustained in mammalian cells such as mouse for a long period of time.

The present inventors have conducted concentrated studies to attain the above object. They used Enhanced Green Fluorescent Protein (EGFP) gene (Cormack et al., 1996) as a heterogenous reporter gene, and constructed a transgene which has inverted repeats of EGFP downstream of a part of the insulator sequence of the chicken beta-globin gene (240 base pairs), a CMV enhancer and human EF1 α promoter, and also has an SV40 poly(A) addition signal. The obtained gene was introduced into a fertilized egg of the EGFP transgenic mouse, and the EGFP fluorescence in the initial generation process was analyzed. As a result, they found that the EGFP fluorescence disappeared even though the generation proceeded normally. The present invention has been completed based on these findings.

Thus, the present invention provides a recombinant gene which contains inverted repeats of a target gene which can be expressed in mammalian cells.

According to preferred embodiments, the present invention provides:
the recombinant gene which contains inverted repeats of a target gene downstream of a promoter sequence which can work in mammalian cells;
the recombinant gene containing an enhancer sequence upstream of a promoter sequence;
the recombinant gene containing an insulator sequence or a part thereof;
the recombinant gene containing a poly(A) addition signal sequence downstream of inverted repeats of a target gene;
the recombinant gene wherein a target gene is a gene of a heterogenous reporter protein or mutant protein thereof; and
the recombinant gene wherein the heterogenous reporter protein is an enhanced green fluorescent protein (EGFP).

The recombinant gene according to the present invention can be preferably used for producing transgenic non-human mammals. Preferably, the non-human mammals is selected from the group consisting of mouse, rat, hamster, guinea pig, rabbit, dog, cat, horse, cattle, sheep, pig, goat, and monkey.

According to another aspect, the present invention provides a recombinant vector containing the above-mentioned recombinant gene of the present invention.

According to further another aspect, the present invention provides a transformant having the above-mentioned recombinant vector.

According to further another aspect, the present invention provides an embryo which is generated from a fertilized egg of a non-human mammals into which the recombinant gene or recombinant vector of the present invention has been introduced.

According to further another aspect, the present invention provides a fetus which is generated by transplanting the embryo into a womb or oviduct of a corresponding non-human mammals.

According to further another aspect, the present invention provides a transgenic non-human mammals, an offspring thereof, or a part thereof, which expresses inverted repeats of a target gene.

According to preferred embodiments, the present invention provides:
the transgenic non-human mammals, an offspring thereof, or a part thereof, having DNA which has been incorporated in such a way that inverted repeats of a target gene can be expressed in a specific site;
the transgenic non-human mammals, an offspring thereof, or a part thereof, having DNA which has been incorporated in such a way that inverted repeats of a target gene can be expressed at a specific stage; and
the transgenic non-human mammals, an offspring thereof, or a part thereof wherein the inverted repeats of a target gene are inverted repeats of a gene of a heterogenous reporter protein or mutant protein thereof.

In the present invention, the non-human mammals is preferably selected from the group consisting of mouse, rat, hamster, guinea pig, rabbit, dog, cat, horse, cattle, sheep, pig, goat, and monkey.

According to still further another aspect, the present invention provides a method for inhibiting the expression of a target gene which comprises introducing a recombinant gene containing inverted repeats of a target gene downstream of an enhancer sequence and a promoter sequence or a recombinant vector containing said recombinant gene, into non-human mammalian cells.

### Brief Description of the Drawings

Fig. 1 is a diagram showing the construction of a plasmid containing an inverted repeats gene of EGFP.
Fig. 2 is a diagram showing the construction of a plasmid containing an inverted repeats gene of EGFP containing 5' DMS240.
Fig. 3 is a diagram showing the construction of a plasmid containing antisense strand EGFP.
Fig. 4 is a diagram showing the structure of a fragment (3.7 kb) of the EGFP dsRNA expression-introduced gene (on its top) and a structure of a fragment (3.0 kb) of the EGFP antisense RNA expression-introduced gene (on its bottom).
Fig. 5 shows the fluorescent image (left) and the visible light image (right) of an embryo after the transgene had been injected into the pronucleus of a fertilized egg.
Fig. 6 shows a mouse which shows decreased EGFP fluorescence on the body surface. 1 to 4 day-old offspring mice were irradiated with an ultraviolet lamp at 365 nm and observed in a dark box. As a result, a mouse which shows decreased EGFP fluorescence on the body surface was observed. In the figure, the mouse at center among 3 offspring mice is the one showing decreased fluorescence.
Fig. 7 shows the EGFP fluorescence observation of lymphocytes of a mouse which shows decreased EGFP fluorescence on the body surface. Mice Nos. HIR-1-16L and HIR-7-238L were found to have RNAi expression. Mice Nos. HIR-1-17L and HIR-7-237L are brood thereof respectively in which the RNAi effect was not observed. B6C3F1 mouse was used as a negative control.
Fig. 8 shows the EGFP fluorescence observation of lymphocytes of a mouse which shows decreased EGFP fluorescence on the body surface (after breeding for a long period of time). The same mouse as used in Fig. 7 was used, and this analysis was carried out 6 months after the analysis shown in Fig. 7.

### Best Mode for Carrying out the Invention

Embodiments of the present invention are described in detail.

The recombinant gene of the present invention comprises inverted repeats of a target gene which can be expressed in mammalian cells. The introduction of a recombinant gene having such a construction into mammalian cells enables the expression of the inverted repeats of a target gene in the cell. This can inhibit the expression of the target gene by the RNA interference (RNAi) effect.

The term "inverted repeats" refers to a sequence in which a target gene is arranged in parallel with an inverted sequence via a suitable sequence. More specifically, when the target gene has a double-strand comprising the following (n) nucleotides,
5'-X₁X₂.....Xₙ₋₁Xₙ-3'
3'-Y₁Y₂.....Yₙ₋₁Yₙ₋5'
the inverted sequence has the following sequence:
5'-YₙYₙ₋₁.....Y₂Y₁-3'
3'-XₙXₙ₋₁.....X₂X₁-5'
wherein the nucleotide represented by X and the nucleotide represented by Y are complementary to each other as long as the numbers represented as subscripts are identical to each other.

The inverted repeats are a sequence comprising the above 2 types of sequences through a suitable sequence. With regards to the construction of the inverted repeats, a sequence of the target gene may be located upstream of the inverted sequence, or an inverted sequence may be located upstream of a sequence of the target gene. The inverted repeats used in the present invention may be either of the above. Preferably, the inverted sequence is located upstream of the sequence of the target gene.

A sequence existing between the sequence of the target gene and the inverted sequence thereof is a region which forms a hairpin loop during RNA transcription. The length of the region is not particularly limited as long as the hairpin loop can be formed. It is generally 0 bp to 700 bp, preferably about 0 to 300 bp, and more preferably about 0 to 100 bp. A restriction site may also be present in this sequence.

Any gene can be used as the target gene used in the present invention. When the recombinant gene of the present invention is used to produce a transgenic animal and gene knock-out caused by RNAi is intended, the target gene is a gene, the expression of which is intended to be inhibited (a gene which is intended to be knocked-out). Examples of such a target gene include a gene which is cloned but whose function is unknown.

Alternatively, the target gene may be the gene of a heterogenous reporter protein or mutant protein thereof. When such a gene of a heterogenous reporter protein or mutant protein thereof is used as a target gene, the RNAi effect can be easily detected and evaluated in the transgenic technique using the recombinant gene of the present invention.

The heterogenous reporter protein includes enhanced green fluorescent protein, green fluorescent protein, aequorin, chloramphenicol acetyltransferase, β -galactosidase, luciferase, and β -glucuronidase.

The mutant protein of the heterogenous reporter protein has substitution, deletion, addition and/or insertion of one to several (for example, I to 20, preferably 1 to 10, and more preferably 1 to 5) amino acids in the amino acid sequence of the wild-type reporter protein. Preferably, the mutant protein maintains a function equivalent to or higher than the wild-type reporter protein.

Specific examples of the gene of the mutant protein of the reporter protein include a gene wherein a portion of the nucleotide sequence is deleted in the gene of the reporter protein, a gene wherein a nucleotide sequence of the reporter gene is substituted with another nucleotide sequence, and a gene wherein another nucleotide sequence is inserted in a part of the nucleotide sequence of the reporter gene. The number of nucleotides subjected to deletion, substitution or addition is not particularly limited, and it is generally about 1 to 60, preferably about 1 to 30, and more preferably about 1 to 10. These mutant genes preferably maintain their functions as reporter genes.

The gene of the mutant protein can be produced by any conventional method in the art, such as chemical synthesis, genetic engineering, or mutagenesis. Specifically, DNA which codes for a native reporter protein is brought into contact with a mutagenic agent, irradiated with ultraviolet, or subjected to genetic engineering techniques such as PCR. Thus, a gene coding for a mutant protein can be obtained. Site-directed mutagenesis, which is one of genetic engineering techniques, is particularly useful since it allows specific mutations to be introduced into specific sites, and it can be carried out in accordance with the method described in Molecular Cloning: A laboratory Manual (2^{nd} ED., Cold Spring Harbor Laboratory, Cold Spring Harbor, NY., 1989), Current Protocols in Molecular Biology (Supplement 1 to 38, John Wiley & Sons (1987-1997)), and the like.

In the recombinant gene of the present invention, inverted repeats of a target gene is present downstream of a promoter sequence which can work in a mammals. Such a construction enables the expression of inverted repeats of the target gene in mammalian cells. More specifically, in the recombinant gene of the present invention, the inverted repeats of the target gene are located so as to be under the control of the promoter described above.

The promoter sequence used in the present invention is not particularly limited as long as it can work in mammals.

Examples of such promoters which can work in non-human animals and can be used herein include: gene promoters derived from viruses (e.g., cytomegalovirus, Moloney leukemia virus, JC virus, and breast cancer virus); and promoters derived from various mammals (e.g., human, rabbit, dog, cat, guinea pig, hamster, rat, and mouse). Promoters derived from various mammals include, for example, promoters of albumin, endothelin, osteocalcin, muscle creatine kinase, collagen I and II, cyclic AMP-dependent protein kinase β subunit (The Journal of Biological Chemistry (vol. 271, No. 3, pp 1638-1644, 1996), atrial natriuretic factor, dopamine β-hydroxylase, neurofilament light chain (The Journal of Biological Chemistry (vol. 270, No. 43, pp 25739-25745, 1995 and vol. 272, No. 40, pp 25112-25120, 1997)), metallothionein, tissue inhibitor of metalloproteinase-1, smooth muscle α-actin, polypeptide chain elongation factor 1α (EF-1α), β-actin, α-myosin heavy chain and β-myosin heavy chain, myosin light chain 1 and myosin light chain 2, myelin base protein, serum amyloid P component, and renin.

In addition to the above promoters, for example, promoters, which are described in the literature such as Molecular Medicine (occasional extra number), "Disease Model Mouse Manual," Ken-ichi Yamamura, Motoya Katsuki, and Shin-ichi Aizawa (ed.), NAKAYAMA SHOTEN CO., LTD. can be used.

Preferable promoters used in the present invention include human EF1 α promoter as is described in the Examples in the present specification, and further include the following.

### (1) β-Actin promoter

It is generally used in combination with the CMV enhancer and the β-actin promoter. Examples include pCAGGS, chicken beta-actin promoter, cytomegalo virus enhancer, beta-actin intron and bovine globin poly-adenylation signal. (see H. Niwa, K. Yamanami, J. Miyazaki, Gene, 108, (1991) 193-199)

### (2) CMV promoter

It is generally used as a combination of the CMV enhancer with the CMV promoter. (see Janet A. Sawicki et al., Experimental Cell Research 244, 367-369 (1998))

### (3) Metallothionein promoter

See "Establishment of Transgenic Mice Carrying Human Fetus-Specific CYP3A7," Yong Li, et al, Archives of Biochemistry and Biophysics, Vol. 329, No. 2, 235-240, 1996

### (4) Apolipoprotein E promoter

A promoter which is intended for expression in fetal liver. (see Simonet et al., 1993, J. Biol. Chem., 268, 8221-8229)

### (5) Promoter inherent to the gene to be introduced

In this case, the genome itself is introduced to produce a transgenic mouse. (see Okamoto M., et al., J. Exp. Med., 175,71 (1992))

The recombinant gene of the present invention may contain an enhancer sequence upstream of the promoter sequence. Enhancer sequences which can be used herein include the above-mentioned CMV enhancer.

The recombinant gene of the present invention may contain an insulator sequence or a portion thereof. "Insulator sequence" refers to a gene sequence, in a transgenic animal, which prevents the inhibition of gene expression resulting from a "position effect". The insulator sequence is expected to be a barrier against the influence of cis-elements existing in the neighborhood.

The position of the insulator sequence or a part thereof is not particularly limited. From the viewpoint of the effect, it is preferably located on the 5' side (upstream) of the transgene (i.e., inverted repeats of a target gene). Most preferably, the insulator sequence or a part thereof is located upstream of the promoter sequence (when the enhance sequence is present, it is located upstream thereof).

In addition to the chicken beta-globin-derived insulator sequence as mentioned in the Examples of the present specification, insulator sequences which can be used in the present invention include, but are not limited to, the following.
(1) scs and scs' sequences of vinegar flies (Rebecca Kellum and Paul Schedl, Cell, Vol. 64, 941-950, March 8, 1991)
(2) Insulator sequence of *Drosophila* gypsy transposon (Holdrige, C., and D. Dorsett, 1991 Mol. Cell. Biol. 11: 1894-1900)
(3) Sea urchin arylsulfatase insulator sequence (Koji Akasaka, et. al., Cellular and Molecular Biology 45 ( 5 ), 555-565,1999)
(4) Human T-cell receptor α/δ locus BEAD element (Zhong, X. P., and M. S. Krangel, 1997 Proc. Natul. Acad. Sci. USA)
(5) Human apolipo-protein B-100 (apoB) matrix attachment site (Namciu et al, 1998, Mol. Cell. Biol. 18: 2382-2391)

The recombinant gene of the present invention may contain a poly(A) addition signal sequence downstream of the inverted repeats of the target gene. Insertion of the poly(A) addition signal sequence enables the termination of transcription of messenger RNA of interest.

Specific examples of poly(A) addition signal sequence include, but are not limited to, the SV40 poly(A) addition signal.

The expression vector of the present invention can be produced by any method known in the art or methods in accordance therewith.

Further, the present invention relates to a recombinant vector containing the recombinant gene of the present invention and a transformant having the recombinant vector.

Specific examples of vectors when a bacterium is used as a host include, but are not limited to, pBTrP2, pBTac1, and pBTac2 (commercially available from Boehringer Mannheim), pKK233-2 (manufactured by Pharmacia), pSE280 (manufactured by Invitrogen), pGEMEX-1 (manufactured by Promega), pQE-8 (manufactured by QIAGEN), pQE-30 (manufactured by QIAGEN), pKYP10 (Japanese Patent Application Laying-Open No. 58-110600), pKYP200 [Agrc. Biol. Chem., 48, 669 (1984)], PLSA1 [Agrc. Blo1. Chem., 53,277 (1989)], pGEL1 [Proc. Natl. Acad. Sci. USA, 82, 4306 (1985)], pBluescrlptII SK+ and pBluescriptII SK(-) (manufactured by Stratagene), pTrS30 (FERMBP-5407), pTrS32 (FERM BP-5408), pGEX (manufactured by Pharmacia), pET-3 (manufactured by Novagen), pTerm2 (US4686191, US4939094, and US5160735), pSupex, pUB110, pTP5, pC194, and pUC18 [Gene, 33, 103 (1985)], pUC19 [Gene, 33, 103 (1985)], pSTV28 (manufactured by Takara Shuzo Co., Ltd.), pSTV29 (manufactured by Takara Shuzo Co., Ltd.), pUC118 (manufactured by Takara Shuzo Co., Ltd.), pPA1 (Japanese Patent Application Laying-Open No. 63-233798), pEG400 [J. Bacteriol., 172, 2392 (1990)], and pQE-30 (manufactured by QIAGEN).

Specific examples of vectors when yeast is used as a host include, but are not limited to, YEp13 (ATCC37115), YEp24 (ATCC37051), Ycp5O (ATCC37419), pHS19, and pHS15.

Specific examples of vectors when an animal cell is used as a host include, but are not limited to, pcDNAI and pcDM8 (commercially available from Funakoshi), pAGE107 [Japanese Patent Application Laying-Open No. 3-22979; Cytotechnology, 3, 133 (1990)], pAS3-3 (Japanese Patent Application Laying-Open No. 2-227075), pCDM8 [Nature, 329, 840 (1987)], pcDNAI/AmP (manufactured by Invitrogen), pREP4 (manufactured by Invitrogen), pAGE103 [J.Blochem., 101, 1307 (1987)], and pAGE210.

In the production of a transformant, any cell can be used as a host as long as the gene of interest can be expressed. Usable examples thereof include: bacteria (for example, *Escherichia, Serratia, Coryncbacterium, Brevibacterium, Pseudomonas, Bacillus, and Mycobacterium),* yeast *(Kluyveromyces, Saccharomyces, schizosaccharomyces, Trichosporon, Schwanniomyces* and the like), animal cells (Namalwa cell, COS1 cell, COS7 cell, CHO cell and the like), plant cells, and insect cells (Sf9 cell, Sf21 cell, High5 cell and the like).

A method for introducing a recombinant vector into a host can be suitably selected according to type of host and the like. Examples of methods for introducing recombinant vectors into bacterial cells include a method employing calcium ion, and the protoplast method. Examples of methods for introducing recombinant vectors into yeast include electroporation method, spheroplast method, and lithium acetate method. Examples of methods for introducing recombinant vectors into animal cells include electroporation method, calcium phosphate method, and lipofection method.

The present invention further relates to an embryo generated from a fertilized egg of a non-human mammals into which the recombinant gene or recombinant vector of the present invention has been introduced, and to a fetus generated by transplanting the embryo into a womb or oviduct of a corresponding non-human mammals. These animals are transgenic non-human mammals which express inverted repeats of a target gene.

Examples of the part of non-human mammals include intracellular organelles, cells, tissues, organs, heads, fingers, hands, legs and feet, abdomens, and tails.

Examples of non-human mammals include, but are not limited to, mouse, rat, hamster, guinea pig, rabbit, dog, cat, horse, cattle, sheep, pig, goat, and monkey. Rodent mammals such as mouse, rat, and guinea pig is preferred as a non-human mammals, with mouse or rat being particularly preferred. Examples of mouse include the lineages of the pure-line C57BL/6, DBA/2, and BALB/c, the lineages of the hybrid-line B6C3F1 and B6D2F1, and the lineage of the closed colony ICR. Specific examples of rat include Wister and SD rats.

In one preferred embodiment, the transgenic non-human mammals of the present invention may incorporate DNA therein in such a way that the inverted repeats of the target gene can be expressed in a specific site.

The phrase "be expressed in a specific site" used herein refers to that the inverted repeats of the target gene can be expressed in a specific location such as intracellular specific sites, intracellular organelles, cells, tissues, or organs.

Intracellular specific sites include the axis cylinder of neurocytes and the like. Intracellular organelles include, for example, nuclei, mitochondria, Golgi apparatuses, endocytoplasmic reticulums, ribosomes, and cell membranes. Examples of cells include: hepatocytes, splenocytes, neurocytes, gliocytes, pancreatic β-cells, myeloma cells, mesangium cells, Langerhans's cells, epidermal cells, epithelial cells, endothelial cells, fibroblasts, fibrocytes, myocytes, adipocytes, immunocytes, megakaryocytes, synoviocytes, chondrocytes, osteocytes, osteoblasts, osteoclasts, alveolar epithelial cells, hepatocytes, or interstitial cells of mammals; and their precursor cells, stem cells, or carcinoma cells. Tissues include any tissue in which the above-mentioned cells are present, for example, brain (e.g., amygdaloid nucleus, basal ganglia, hippocampus, hypothalamus, cerebral cortex, medulla, cerebellum, and epiphysis), spinal cord, hypophysis, stomach, sexual gland, thyroid gland, gallbladder, bone marrow, adrenal gland, skin, muscle, lung, large intestine, small intestine, duodenum, rectum, blood vessel, thymus gland, submandibular gland, peripheral blood, prostate gland, spermary, ovary, placenta, uterus, bone, articulation, and skeletal muscle. Alternatively, it may be a hemocyte or a cultured cell of the above-mentioned cells. Organs include heart, kidney, pancreas, liver, and spleen.

According to one preferred embodiment, the transgenic non-human mammals of the present invention may incorporate DNA therein in such a way that the inverted repeats of the target gene can be expressed at specific stages.

The term "specific stages" used herein refers to specific stages from embryo generation, birth, generation up to death. Accordingly, a specific stage may be any of each stage during embryo generation including a stage of the introduction of the heterogenous gene, on the basis of hours, days, weeks, months, and years being passed after birth.

In order to express the inverted repeats of the target gene at a specific stage in the transgenic non-human mammals of the present invention, an expression vector which contains a promoter region capable of expressing a protein at specific stages, and a signal sequence capable of expressing a protein at specific stages or the like is used to produce a transgenic non-human mammals having inverted repeats of a target gene.

The inverted repeats of the target gene can also be expressed at specific stages by constructing a system for the induction of protein expression or by administering a protein expression inducer to a non-human mammals at specific stages. Examples of the protein expression-inducing system usable herein include an expression induction system which utilizes tetracyclin or ecdysone. The agent which is administered in that case is tetracycline or an analogue thereof or ecdysone or an analogue thereof, respectively. Also, the Cre-loxP system utilizing a recombinase and the like may be employed.

Further, the inverted repeats of the target gene can be expressed at specific stages by incorporating any resistant gene against antibiotics such as tetracyclin, kanamycin, hygromycin or puromycin into an expression vector. The location of the resistant gene in the expression vector according to the present invention is not particularly limited. In general, it is preferably located downstream of the reporter gene or mutant gene thereof, or upstream of the promoter region or signal sequence.

The transgenic non-human mammals of the present invention can be produced by introducing a recombinant gene containing inverted repeats of the target gene (hereinafter this may be referred to as a "transgene") downstream of the promoter sequence which can work in mammalian cells, into an object animal. More specifically, by introducing the transgene into a fertilized egg, embryonic stem cell, somatic cell, sperm, or unfertilized egg of a non-human mammals of interest, a transgenic non-human mammals having the gene incorporated into the chromosomes of all the cell including germinal cells can be obtained. Introduction of transgene into a fertilized egg, embryonic stem cell, somatic cell, sperm, or unfertilized egg should be carried out in such a way that the gene is present in the chromosomes of all the cells including germinal and somatic cells of the non-human mammals of interest.

Offsprings of the transgenic non-human mammals wherein the transgene is integrated in the chromosomes of all the cells including germinal similarly possess the gene of interest. Homozygote animals having the transgene in both of the homologous chromosomes are obtained, and female and male thereof were mated in such a manner that all offspring stably sustain the gene, thus confirming the possession of the genes. Thereby, the offspring can be reproduced and subcultured in a general breeding environment.

A method for producing the transgenic non-human mammals of the present invention is described below in more detail.

The transgenic non-human mammals of the present invention can be produced by introducing the transgene into, for example, a fertilized egg, and sperm and a germ cell containing a progenitor cell thereof, preferably at the early stage of embryo formation (more preferably at the single cell stage or amphicytula and before the 8-cell stage in general) in the generation of non-human mammals.

The construction and the method for constructing the transgene are described above in the present specification.

The fertilized egg to be used when introducing the transgene into the fertilized egg of the non-human mammals of interest or its progenitor is obtained by mating a male non-human mammals with a conspecific female. The fertilized egg can be obtained by natural mating, however, it is preferably obtained by artificially controlling the estrous cycle of the female non-human mammals and then mating it with a counterpart male. A preferred method for artificially controlling the estrous cycle of the female non-human mammals is carried out, for example, by first administering follicle stimulating hormone (pregnant mare serum gonadotropin) and then administering luteinizing hormone (human chorionic gonadotropin) by abdominal injection or the like. Preferably, the dose and the administration interval of hormone can be suitably determined based on the type of mammals.

Examples of methods for introducing a transgene include conventional methods such as Calcium phosphate method, electropulse method, lipofection method, coagulation method, microinjection method, particle gun method, and DEAE-dextran method. The transgene of interest is introduced into a somatic cell in accordance with the above mentioned introduction method, and this cell (or a nucleus thereof) is fused with the above germ cell in accordance with any known cell fusion technique. Thus, the transgenic non-human mammals of the present invention can be produced.

The construction and the method for constructing the transgene are described above in the present specification. The introduction of a transgene at the stage of amphicytula is carried out in such a way that the transgene is present in all germ and somatic cells of the subject animal.

After the transgene is introduced into the fertilized egg, the egg is artificially transplanted and implanted into a female non-human mammals. Thus, the transgenic non-human mammals having the transgene can be obtained. It is preferable that luteinizing hormone releasing hormone (LHRH) or an analogue thereof is administered, and then the female non-human mammals is mated with a male mammals, and thereby the transgene-introduced fertilized egg is artificially transplanted and implanted into a pseudopregnant female non-human mammalian species. The dose of LHRH or an analogue thereof and the stage of mating with a conspecific male after the administration can be suitably selected depending on the type of non-human mammals and the like.

Whether or not the transgene is successfully incorporated into the genomic DNA can be confirmed by extracting DNA from the tail of the offspring and examining by the Southern hybridization, PCR or the like.

If the transgene is present in the germ cell of the produced animal after the introduction of transgene, it indicates that the progeny of the produced animal maintains the transgene in all the germ cells and somatic cells. The offspring animal of this species, which inherited the transgene, has the transgene in all the germ cells and somatic cells.

A homozygote animal having the transgene in both homologous chromosomes is obtained, and the female is mated with the male, thereby reproducing and subculturing in such a manner that all the offspring have the transgene. The thus obtained offspring is also included in the animal according to the present invention.

Regarding the detailed production process of the transgenic animals, reference can be made to, for example, Manipulating the Mouse Embryo (Brigid Hogan et al, Cold Spring Harbor Laboratory Press, 1986), Gene Targeting, A Practical Approach (IRL Press at Oxford University Press (1993)), Bio Manual Series 8, Gene Targeting (Production of Mutant Mouse using ES Cell, YODOSHA CO., LTD. (1995)), and Experimental Manual for Development Engineering, A Method for Producing Transgenic Mouse (Kodansha Ltd. (1987)).

In the transgenic non-human mammals which expresses inverted repeats of the target gene according to the present invention, the expression of the target gene is expected to be inhibited by the RNAi effect. Specifically, a method for inhibiting the expression of the target gene which comprises introducing a recombinant gene containing inverted repeats of a target gene downstream of an enhancer sequence and a promoter sequence or a recombinant vector containing the recombinant gene into the cell of the non-human mammals, is within the scope of the present invention.

An animal model in which the function of the target gene is knocked-out, is useful for analyzing the function of the novel gene.

All of the contents disclosed in the specification of Japanese Patent Application No. 2001-46089, based on which the present application claims priority, is incorporated herein by reference as a part of the disclosure of the present application.

The present invention will be described in more detail with reference to the following examples, but the present invention is not limited to these examples.

### Examples

### Example 1: Construction of transgene 5' INS 240 CE EGFP IR

In the early stage of the study of insulator, the transgene was produced from a vector having the same insulator sequences of 240 nucleotides on each of the 5' side and the 3' side of the cloning site. Thereafter, the result obtained from the experiment on the model suggested that it was most effective when the insulator sequence was inserted only into the 5' side of the transgene. Accordingly, the 3' insulator sequence which has been inserted into the 3' terminus of the transgene, was removed from pUC19 5'3'INS240 CE EGFP IR having the same insulator sequences of 240 nucleotides on the each of the 5' side and the 3' side, and the EGFP inverted repeats downstream of the CMV enhancer and the EF1α promoter. Thus, the transgene, 5'INS 240 CE EGFP IR, was constructed as described below.

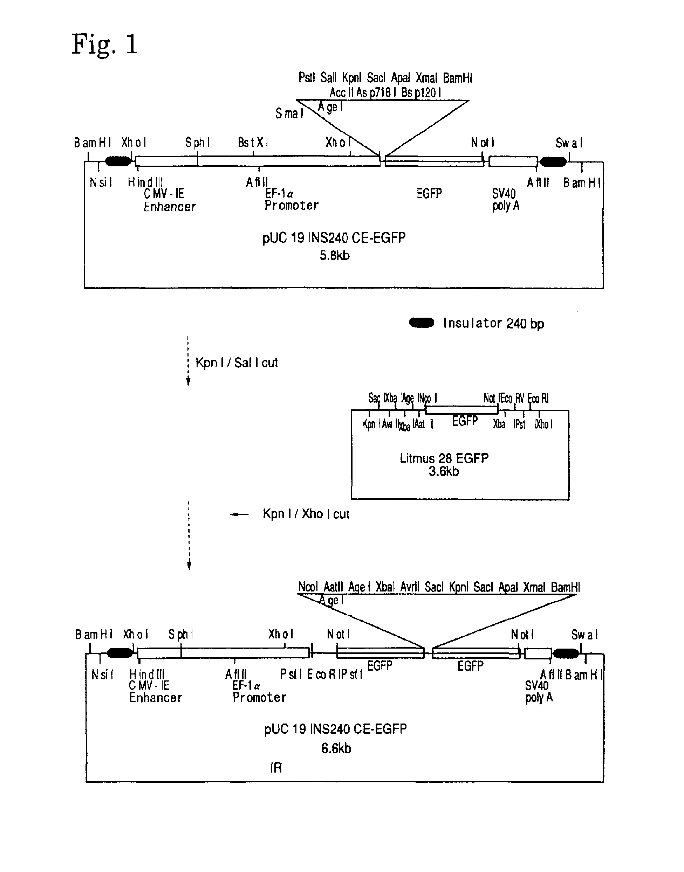

A fragment between *Xho*I and *Afl*II of pCE-EGFP-1 (the name of literature: Takada, T. et al., Selective production of transgenic mice using green fluorescent protein as a marker. Nature Biotech. 15: 458-461, 1997) was inserted into the *Xho*I and *Afl*II sites of pUC19 5', 3' INS240 (a vector constructed by chemically synthesizing 10 partial fragments of the gene of a chicken beta globin-derived insulator sequence, ligating them by DNA ligase to prepare a fragment of 240 base pairs, and inserting the fragment into the multicloning site of the pUC19 vector) at two steps and ligated, and *E. coli* JM109 strain was transformed, thereby obtaining plasmid pUC19 5', 3' INS240 CE EGFP (Fig. 1).

The *Kpn*I*-Xho*I fragment of the Litmus28 EGFP was inserted into the *Kpn*I and *Sal*I cleavage sites of pUC19 5', 3' INS240 CE EGFP, followed by ligation. *E. coli* SURE2 strain (Stratagene) was transformed, and plasmid pUC19 5',3'INS240 CE EGFP IR having inverted repeats was obtained (Fig. 1).

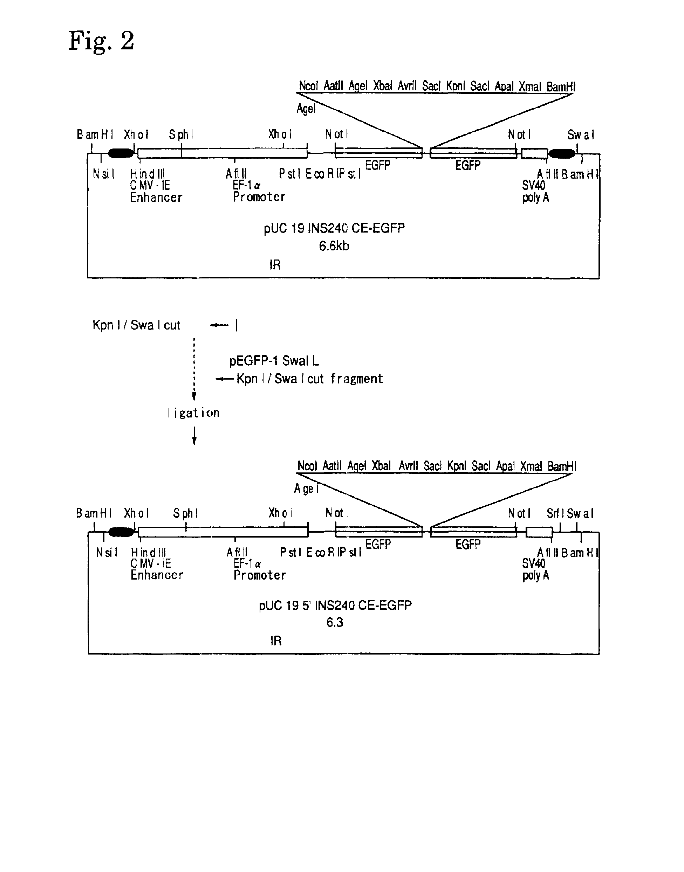

A fragment containing the 3' insulator sequence was cleaved out of pUC19 5',3'INS240 CE EGFP IR as described below (Fig. 2).

pUC19 5',3'INS240 CE EGFP IR was treated with *Kpn*I and *Swa*I. By about 5.4 kbp electrophoresed DNA fragment, it was confirmed that the presence of pUC19 5',3'INS240 CE EGFP IR/*Kpn*I, *Swa*I vector. Further, in order to prevent self-ligation, dephosphorylation by BAP was carried out. Thereafter, phenol extraction, chloroform extraction, and ethanol precipitation were carried out to remove BAP.

Plasmid pEGFP-1 SwL, which was constructed by inserting a linker having a *Swa*I restriction site, into the *Afl*II restriction site of pEGFP-1 (Clontech), is treated with *Kpn*I and *Swa*I and then subjected to agarose gel electrophoresis, and about 1.0 kbp DNA fragment was cleaved out to obtain *Kpn*I*-Swa*I fragment (Fig. 2).

The pUC19 5',3'INS240 CE EGFP IR/*Kpn*I, *Swa*I vector was ligated to the *KpnI-SwaI* fragment using DNA Ligation Kit ver. 2 (Takara), *E. coli* SURE2 strain was transformed, and a positive clone was obtained by screening based on the length of the DNA fragment obtained by treatment with *Eco*T22I and *Swa*I, *Not*I*.* After the DNA sequence was confirmed, it was designated as "pUC19 5'INS240 CE EGFP IR"

As a control for the inverted repeats, a transgene having the EGFP antisense sequence downstream of a similar insulator, the CMV enhancer, and the EF-1 α promoter was constructed. The control gene was constructed through the removal of the IR sequence from pUC19 5'INS240 CE EGFP IR, the introduction of the *Kpn*I restriction site, and the introduction of the EGFP antisense strand into between *Eco*RI and *Kpn*I*.* The method therefor is described below.

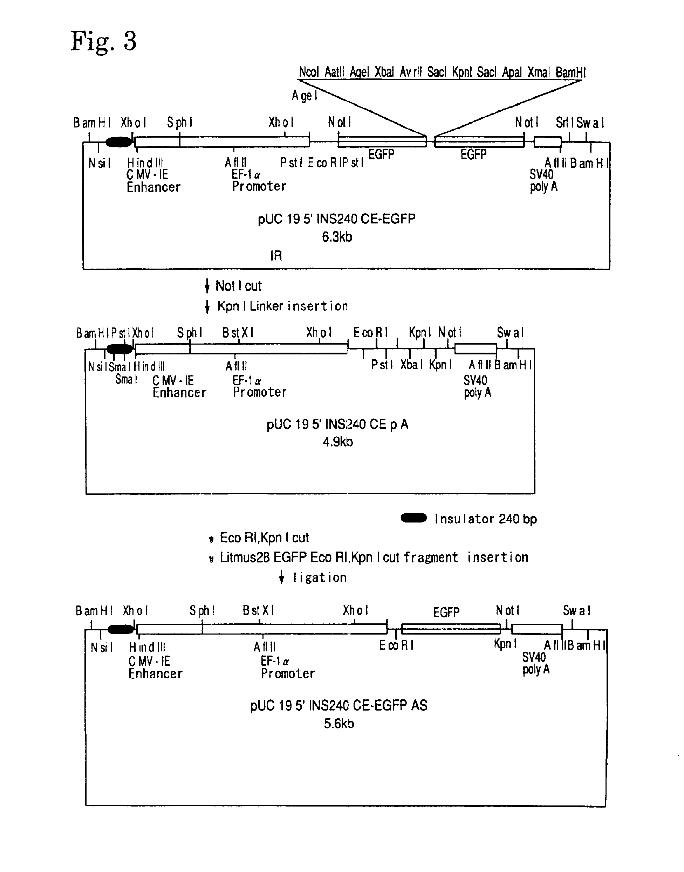

At first, the gene fragment of the EGFP inverted repeats was cleaved and the *Kpn*I linker was inserted in the following manner (Fig. 3).

Plasmid DNA, pUC19 5'INS 240 CE EGFP IR was treated with *Not*I to obtain pUC19 5'INS 240 CE EGFP IR/*Not*I vector. Further, in order to prevent self-ligation, dephosphorylation by CIAP, followed by purification through phenol extraction, chloroform extraction, and ethanol precipitation were carried out.

Using DNA Ligation Kit ver. 2 (Takara), the pUC19 5'INS 240 CE EGFP IR/*Not*I vector was ligated to the linker *Kpn*I Linker which has a *Kpn*I restriction site, and *E. coli* JM109 strain was transformed. Thus, plasmid pUC19 5'INS240 CE KpL having the *Kpn*I site therein was obtained.

Subsequently, pUC19 5'INS240 CE KpL was treated with *Eco*RI and *Kpn*I, a fragment, which was subjected to agarose gel electrophoresis, was then cleaved out and recovered, and the recovered fragment was designated as the pUC19 5'INS240 CE KpL/*Eco*RI,*Kpn*I vector. Further, in order to prevent self-ligation in the subsequent operation, dephosphorylation by CIAP was carried out, followed by phenol extraction, chloroform extraction, and ethanol precipitation to remove CIAP.

LITMUS28 EGFP, which was constructed by inserting the EGFP marker gene into Litmus 28, was treated with *Eco*RI and *Kpn*I. The product was subjected to agarose gel electrophoresis to obtain a DNA fragment of LITMUS28 EGFP/*Eco*RI-*Kpn*I (Fig. 3).

Using DNA Ligation Kit ver. 2, the pUC19 5'INS240 CE KpL/*Eco*RI-*Kpn*I vector was ligated to the LITMUS28 EGFP/*Eco*RI*-Kpn*I EGFP fragment, and *E*. *coli* JM109 strain was transformed. Then, a positive clone was obtained by screening based on the length of the fragment which was treated with restriction enzymes *Bsr*GI and *Swa*I. The sequence was confirmed by sequencing. This plasmid was designated as "pUC19 5'INS240 CE EGFP AS."

### Example 2: Preparation of transgene fragment

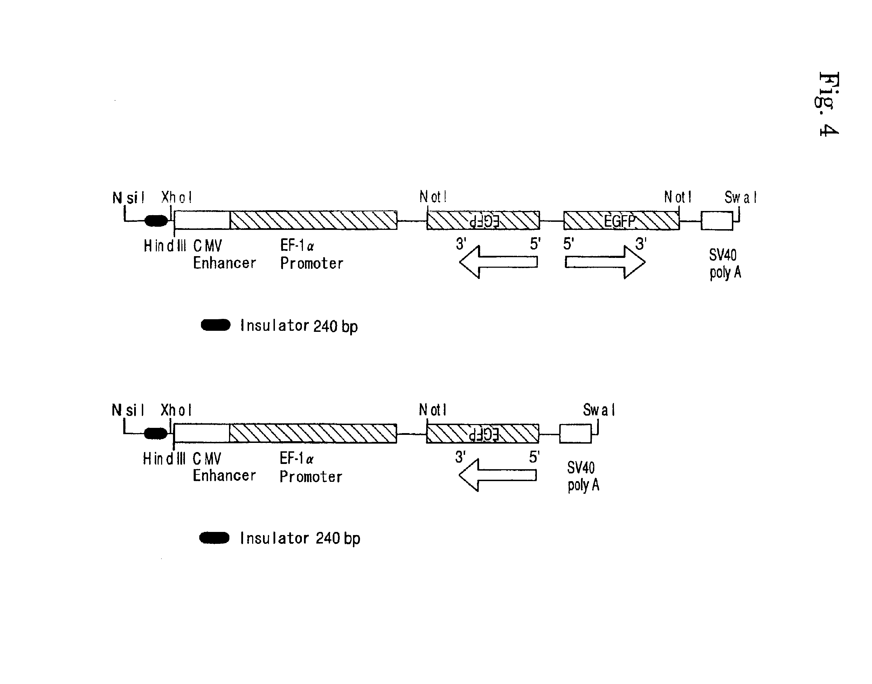

### (1) Preparation of gene fragment of EGFP inverted repeats (Figure on the top in Fig. 4)

pUC19 5'INS240 CE EGFP IR plasmid DNA was prepared in a large scale by the alkali lysis method. This plasmid was treated with *Eco*T22I and *Swa*I, and about 3.7 kbp DNA fragment was separated by agarose gel electrophoresis, and recovered by electroelution. Further, the product was purified by phenol/chloroform extraction, chloroform extraction and ethanol precipitation. The purified product was further purified by ultracentrifugation, followed by dilution with PBS(-) to 1.5 ng/ml. This solution was filtered through a 0.22 *µ*m filter to obtain a transgene fragment.

### (2) Preparation of gene fragment of EGFP antisense sequence (Figure on the bottom in Fig. 4)

pUC19 5'INS240 CE EGFP AS plasmid DNA was prepared in a large scale by the alkali lysis method. This plasmid was treated with *Eco*T22I and *Swa*I, and about 2.9 kbp DNA fragment was separated by agarose gel electrophoresis and recovered by electroelution. Further, the product was purified by phenol/chloroform extraction, chloroform extraction and ethanol precipitation. The purified product was further purified by ultracentrifugation, followed by dilution with PBS(-) to 1.2 ng/ml. This solution was filtered through a 0.22 *µ*m filter to obtain a transgene fragment.

### Example 3: Examination of expression of RNAi effect in early mouse embryo

The expression of RNAi effect in early mouse embryo was examined in the following manner.

### (1) Production of fertilized egg

The fertilized egg was produced by *in-vitro* fertilization in accordance with the technique by Toyoda et al. (1971). Specifically, PMSG and hCG (5 units) were administered to a female mouse through intraperitoneal injection at 48 hour intervals. Thereafter, eggs were collected 16 to 18 hours later, and were inseminated (about 100 to 150 sperms/*µ*l) with sperm of the EGFP transgenic mouse (Masaru Okabe et al., FEBS Letters 407 (1997) 313-319) (sperm was collected about 1.5 hour before egg collection). About 6 hours after insemination, discharge of the secondary polar body of egg and the presence of both pronucleus of male and female were confirmed, and only the fertilized egg was selected. The obtained fertilized eggs at the pronucleus stage were cryopreserved by simple vitrification in accordance with the technique by Nakao et al. (1997). The frozen fertilized eggs were melted before the experiment and subjected to microinjection.

### (2) Microinjection of transgene

Microinjection of the transgene into the pronucleus of the fertilized egg was carried out in accordance with the technique by Katsuki et al. (1987). The fertilized eggs were transferred to drops of modified Whitten's medium (mWM medium). Regarding injection into the nucleus, after the male pronucleus was confirmed under the phase-contrast microscope (Invert Scope D: Ziess), about 2 pl of the purified DNA solution was injected. Regarding injection into cytoplasm, about 2 pl of the DNA solution was injected into the cytoplasm. The surviving embryos were transferred into the mWM medium, and further subjected to explant culturing under 5% CO₂, 95% Air, and 37°C.

### (3) Observation of EGFP expression in embryo in process of generation

Embryos from each generation stage were transferred into the mWH medium, and the expression of EGFP was then observed using a fluorescent stereoscopic microscope (MZ FL III, Leica), followed by photographing.

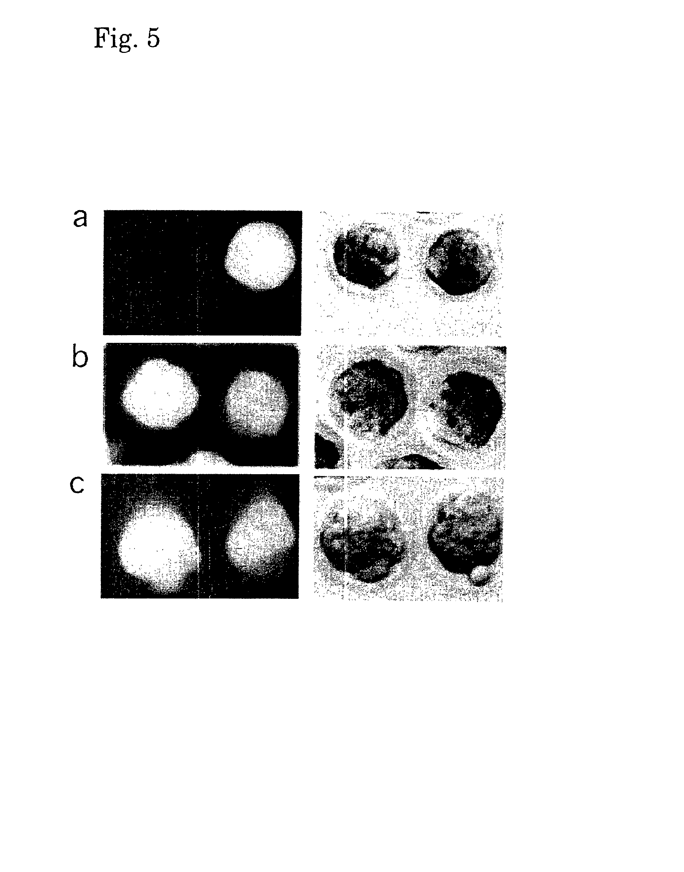

The fluorescent image (left) and the visible light image (right) of the embryo 3 days after the injection of the transgene into the pronucleus of the fertilized egg are shown in Fig. 5.

In this case, the generation stage of the embryo was "morula." In Fig. 5, "a" represents a gene fragment of EGFP inverted repeats, "b" represents a pronucleus into which the gene fragment of the EGFP antisense sequence has been injected, and "c" represents a pronucleus into which DNA was not injected.

In the embryo, the observation of EGFP fluorescence was started from 2 days after the generation. In the embryo into which the gene fragment of the EGFP inverted repeats has been injected, disappearance of EGFP fluorescence was observed ("a" in Fig. 5).

### Example 4: Examination of expression of RNAi effect in an individual mouse

The expression of RNAi effect in an individual mouse was examined in the following manner.

### (1) Production of fertilized egg and microinjection of transgene

Fertilized egg was produced and microinjected with the transgene in the same manner as in Example 3 except that the phase-contrast microscope (DMIRB: Leica) was used. Specifically, 2 pl of purified DNA solution was injected into the pronucleus of the fertilized eggs which were obtained using the sperm of the EGFP transgenic mouse.

### (2) Examination of expression of RNAi effect in an individual mouse

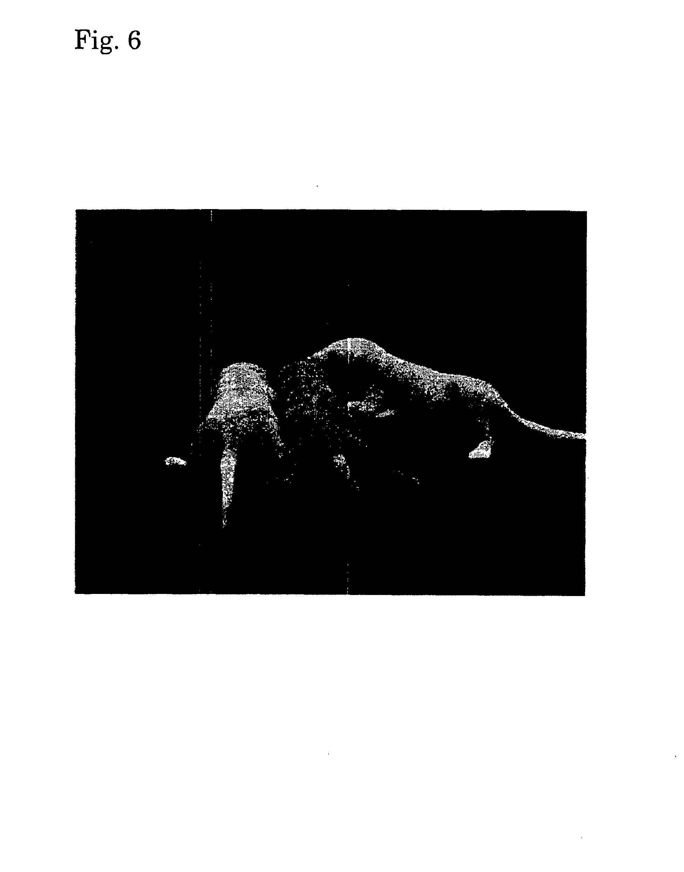

Microinjected fertilized eggs were transferred to the mWM medium, cultured overnight under 5% CO₂, 95% Air, at 37°C, and transplanted into the oviduct of pseudopregnant female mouse of ICR lineage at the 2-cell stage. The female mouse was subjected to cesarean section 18 days later, and offspring mice were obtained. In a dark box (UVP, Model C-10), the obtained offspring mice were irradiated with ultraviolet lamp at 365 nm (UVP, Model UVL-56), and the EGFP fluorescence was observed. Among the offspring mice obtained from fertilized eggs microinjected with EGFP dsRNA expression gene, one mouse which shows decreased EGFP fluorescence on its body surface was visually observed (Fig. 6, in order to remove the blue haze resulting from long wave ultraviolet, safety eyeglasses for ultraviolet was used as a filter, and photographing was carried out using a digital video camera (Panasonic)). In contrast, among the offspring mice obtained from fertilized eggs microinjected with the antisense RNA expression gene, no offspring mouse which shows decreased EGFP fluorescence was observed.

### (3) Analysis of mouse expressing RNAi effect

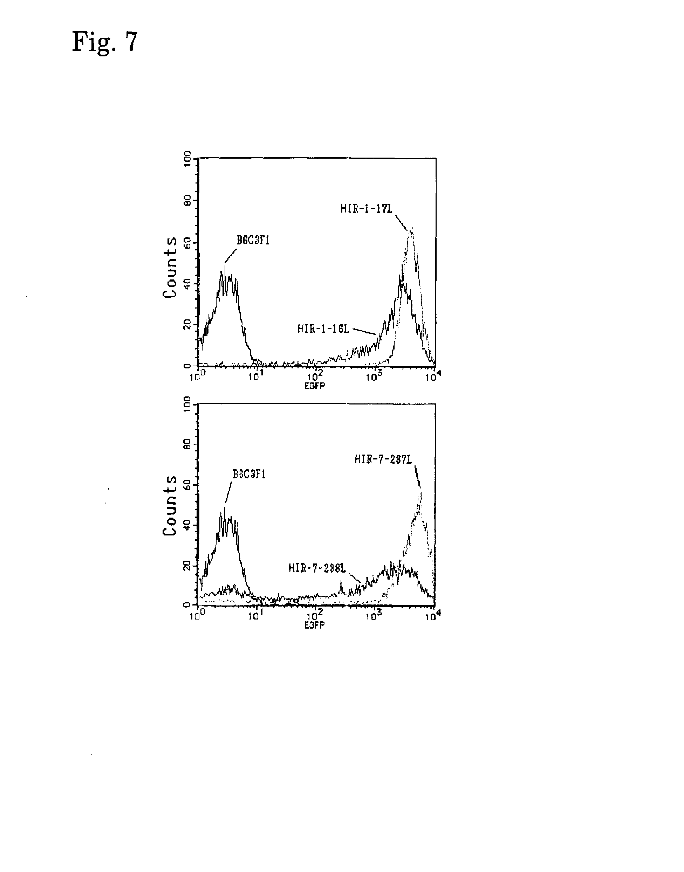

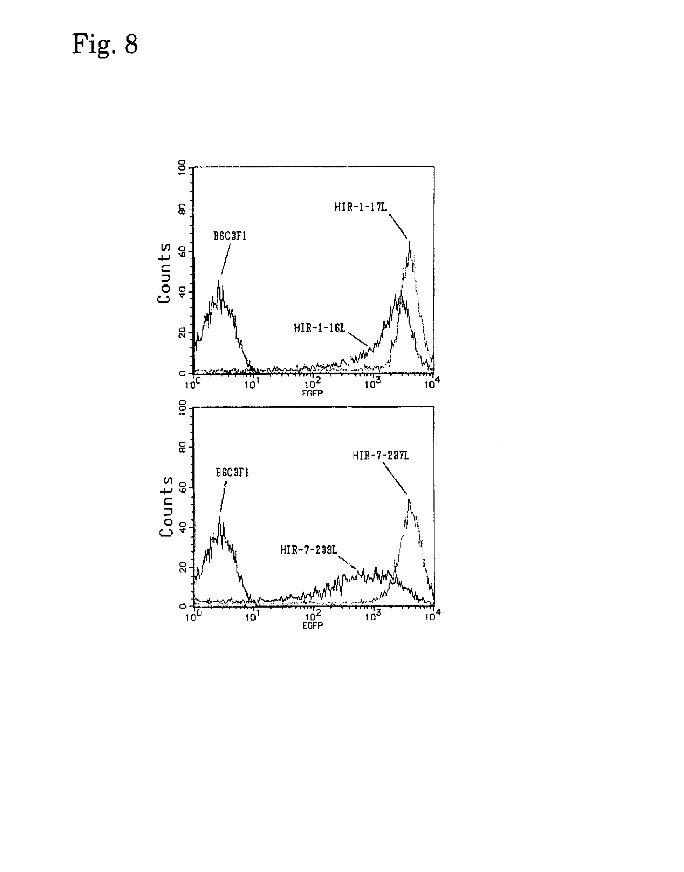

The offspring mice were bred under SPF conditions. 3-week old or older mice were subjected to partial blood sampling from the orbital venous plexus using a heparin-deposited blood-collecting vessel, and peripheral blood mononucleocyte was separated using Lympholyte M (Cedarlane) and analyzed using FACScan (BD). As a result, a group of lymphocytes showing decreased EGFP fluorescence, was observed (Fig. 7). This decrease in the EGFP fluorescence was also observed in mouse lymphocytes after being bred for a long period of time (Fig. 8).

### Industrial Applicability

Accordance to the technique of the present invention, when the gene function of a novel gene is analyzed in a fetus or an individual experimental animal, the result can be obtained faster than the conventional knock-out technique. Thus, it is industrially useful in the analysis of genetic and genetic-related diseases and the like, and in the analysis of a target gene for pharmaceuticals, and the like.

## Claims

1. A recombinant gene which contains inverted repeats of a target gene which can be expressed in mammalian cells.

2. The recombinant gene according to claim 1, which contains inverted repeats of a target gene downstream of a promoter sequence which can work in mammalian cells.

3. The recombinant gene according to claim 1 or 2, which contains an enhancer sequence upstream of a promoter sequence.

4. The recombinant gene according to any of claims 1 to 3, which contains an insulator sequence or a part thereof.

5. The recombinant gene according to any of claims 1 to 4, which contains a poly(A) addition signal sequence downstream of inverted repeats of a target gene.

6. The recombinant gene according to any of claims 1 to 5, wherein a target gene is a gene of a heterogenous reporter protein or mutant protein thereof.

7. The recombinant gene according to claim 6 wherein the heterogenous reporter protein is an enhanced green fluorescent protein (EGFP).

8. The recombinant gene according to any of claims 1 to 7, which is used for producing transgenic non-human mammals.

9. The recombinant gene according to claim 8, wherein the non-human mammals is selected from the group consisting of mouse, rat, hamster, guinea pig, rabbit, dog, cat, horse, cattle, sheep, pig, goat, and monkey.

10. A recombinant vector containing the recombinant gene of any of claims 1 to 9.

11. A transformant having the recombinant vector of claim 10.

12. An embryo which is generated from a fertilized egg of a non-human mammals into which the recombinant gene of any of claims 1 to 9 or recombinant vector of claim 10 has been introduced.

13. A fetus which is generated by transplanting the embryo of claim 12 into a womb or oviduct of a corresponding non-human mammals.

14. A transgenic non-human mammals, an offspring thereof, or a part thereof, which expresses inverted repeats of a target gene.

15. The transgenic non-human mammals, an offspring thereof, or a part thereof according to claims 14, which has DNA which has been incorporated in such a way that inverted repeats of a target gene can be expressed in a specific site.

16. The transgenic non-human mammals, an offspring thereof, or a part thereof according to claims 14 or 15, which has DNA which has been incorporated in such a way that inverted repeats of a target gene can be expressed at a specific stage.

17. The transgenic non-human mammals, an offspring thereof, or a part thereof according to any of claims 14 to 16, wherein the inverted repeats of a target gene are inverted repeats of a gene of a heterogenous reporter protein or mutant protein thereof.

18. The transgenic non-human mammals, an offspring thereof, or a part thereof according to any of claims 14 to 17, wherein the non-human mammals is selected from the group consisting of mouse, rat, hamster, guinea pig, rabbit, dog, cat, horse, cattle, sheep, pig, goat, and monkey.

19. A method for inhibiting the expression of a target gene which comprises introducing a recombinant gene containing inverted repeats of a target gene downstream of an enhancer sequence and a promoter sequence or a recombinant vector containing said recombinant gene, into non-human mammalian cells.
